Europäisches Patentamt

European Patent Office

Office européen des brevets

(11) Publication number: **0 435 506 A2**

# EUROPEAN PATENT APPLICATION

(21) Application number: 90313432.8

(51) Int. Cl.5: **A61N 5/06**

(22) Date of filing: **11.12.90**

(30) Priority: **19.12.89 US 452899**

(43) Date of publication of application:
**03.07.91 Bulletin 91/27**

(84) Designated Contracting States:
**AT BE CH DE DK ES FR GB GR IT LI LU NL SE**

(71) Applicant: **Pfizer Hospital Products Group, Inc.**
**235 East 42nd Street**
**New York New York 10017(US)**

(72) Inventor: **Freiberg, Robert J.**
**23492 Campestre**
**Mission Viejo, California(US)**

(74) Representative: **Wood, David John et al**
**PFIZER LIMITED, Ramsgate Road**
**Sandwich, Kent CT13 9NJ(GB)**

(54) Infrared delivery system with aiming component.

(57) A delivery system including an aiming component for defining a target area and for illuminating the area being contacted by infrared radiation. The system might comprise a medical delivery system (10) which might include a medical instrument, particularly a flexible laser catheter (24), wherein the aiming component is used to illuminate a tissue site being subjected to infrared therapeutic radiant energy.

EP 0 435 506 A2

## INFRARED DELIVERY SYSTEM WITH AIMING COMPONENT

The present invention pertains generally to energy delivery systems which operate in the infrared. More particularly, the invention relates to providing a visible aiming component for systems delivering infrared radiation via optical fibers and waveguides wherein visible light is conveyed to illuminate a target area subjected to infrared radiant energy.

There exist in the art devices used for transmission of infrared energy to a work site. Many of these devices particularly those devices used for medically treating a patient, employ some type of optical system to guide the energy to the work or operating location. Often the devices employ an articulated arm, a combination articulated arm and flexible fiber waveguide, or an operating room microscope interfaced with an infrared laser beam and a superimposed visible aiming beam.

Typically, an articulated arm having a suitable number of tubular segments rotatably coupled to one another and having mirrors provided at the joints of coupled segments, has been employed to direct an infrared operating beam. The beam is transmitted from one tubular segment to the next segment via repeated reflection by the mirrors. Since the operating beam is in the infrared and cannot be seen, typically, a visible guiding light beam is superimposed on the operating beam and both the visible and infrared beams are directed coincidently through the articulated arm by mirror reflection. Alternatively, an optical fiber is used for transmission of the visible guiding light beam while the articulated arm is used to transmit the operating beam and the beams, via mirrors, are coincidently delivered to the operating site.

Another device employs a first optical fiber pathway used for the transmission of infrared laser rays, a second optical fiber pathway used for the transmission of visible rays, and a converging lens system at the distal end of the first and second pathways for focusing the infrared and visible rays delivered to the operating location. Yet another device employs a rotating prism to combine a visible marking beam and an infrared beam. Still another device utilizes a number of discreet fiber optic bundles, spaced apart, with one bundle used to transmit visible light, another bundle to reflect visible light, and a third bundle to deliver infrared radiant energy.

Considerable effort has been devoted to the development of flexible fiber optic delivery systems which will efficiently transmit infrared radiation. In many instances, particularly medical applications where an infrared surgical laser is employed, it is essential that a visible aiming or marking beam be provided for safety considerations. Often, materials used in optical delivery systems are suitable for the longer wavelength infrared radiation but do not provide efficient transmission of visible light. Lack of efficient transmission of visible light becomes even more acute when an optical delivery system is mechanically bent in an arc.

There are many cases in which a visible aiming beam cannot be transmitted along a common pathway travelled by the infrared beam. This is particularly true in medical devices wherein visible light and therapeutic radiation often travel distinct pathways. Therefore, an axiliary means of providing infrared delivery systems with an aiming component must be employed. Accordingly, herein disclosed is such a system which is especially useful in the medical arts field and, most appropriately, in a laser catheter delivery system. The laser catheter disclosed, which will be subjected to bending into a number of short radius of curvature arcs during usage, is very flexible and it includes a flexible infrared delivery component and a flexible aiming component, the latter minimizing loss of transmitted visible light.

The present invention is directed toward a system for transmitting energy to a work site comprising a first energy source for emitting infrared radiation; a second energy source for emitting visible light; first means operatively associated with the first energy source for conveying the radiation to the work site; and second means operatively associated with the second energy source for conveying the visible light to the work site, the second means being disposed peripherally about the first means, with the visible light adapted to illuminate the work site contacted by the radiation.

Embodied within the invention is a medical system for transmitting energy to a tissue site and wherein the first energy source emits infrared therapeutic radiation. The first energy conveying means comprises at least one optical fiber, which might either be a solid core optical fiber or a hollow core optical fiber, having a diameter in a range of from about 200 to about 600 microns. Alternatively, the first means might comprise a metal waveguide. The second energy conveying means might be a sleeve, at least one optical fiber or at least one elongated rod. When a plurality of second means fibers or rods are used, they are oriented substantially parallel to one another. The fibers of the second means might be disposed either in a generally coil-like configuration about the first means or oriented longitudinally about the first means. The elongated rods are preferably oriented longitudinally about the first means. Preferably, the second means fibers and rods have a diameter in a range of from about 10 to about 100 microns.

The invention additionally embodies a medical instrument for transmitting energy to a tissue site comprising a flexible laser catheter adapted to be operatively coupled to a first energy source for emitting infrared laser radiation and to a second energy source for emitting visible light, the catheter having a body housing first means operatively associated with the first energy source for conveying the laser radiation to the tissue site, and second means operatively associated with the second energy source for conveying the visible light to the tissue site, the second means being disposed peripherally about the first means, with the visible light adapted to illuminate the tissue site contacted by the laser radiation. In one embodiment, each of the first and second means comprises at least one flexible optical fiber. The fiber of the second means might be wrapped about the fiber of the first means in a generally coil-like configuration. When the second means comprises a plurality of optical fibers, the fibers are oriented substantially parallel to one another. In another embodiment, the first means comprises at least one flexible optical fiber and the second means comprises one or more flexible rods. When the second means comprises a plurality of rods, the rods are oriented substantially parallel to one another. In a further embodiment, the first means comprises at least one flexible optical fiber and the second means comprises a light transmissive sleeve. In yet another embodiment, the first means comprises a metal waveguide and the second means might comprise a light transmissive sleeve, at least one flexible optical fiber, or one or more flexible elongated rods. When the second means comprises a plurality of fibers or rods, the fibers or rods are oriented substantially parallel to one another. The fiber might either be wrapped about the waveguide in a generally coil-like configuration or longitudinally disposed about the periphery of the waveguide, while the rod, preferably, is disposed longitudinally about the waveguide perimeter.

Fig. 1 is a schematic representation of a medical laser system.

Fig. 2 is a schematic representation of a flexible laser catheter, much like that depicted in Fig. 1, but illustrating the catheter in greater detail, particularly the end portions thereof.

Fig. 3 is an enlarged, detailed, partial sectional view of a first embodiment of an end portion of the catheter of Fig. 2 and further schematically showing a source of visible light and its path of transmission.

Fig. 3A is an end elevational view of the right end of Fig. 3.

Figs. 3B and 3C are end elevational views like that of Fig. 3A but depicting modified end configurations.

Fig. 4 is an enlarged schematic partial view of a second embodiment of an end portion of the catheter of Fig. 2 and further schematically showing a source of visible light and its path of transmission.

Fig. 4A is an end elevational view of the right end of Fig. 4.

Fig. 5 is an enlarged schematic partial view of a third embodiment of an end portion of the catheter of Fig. 2 and further schematically showing a source of visible light and its path of transmission.

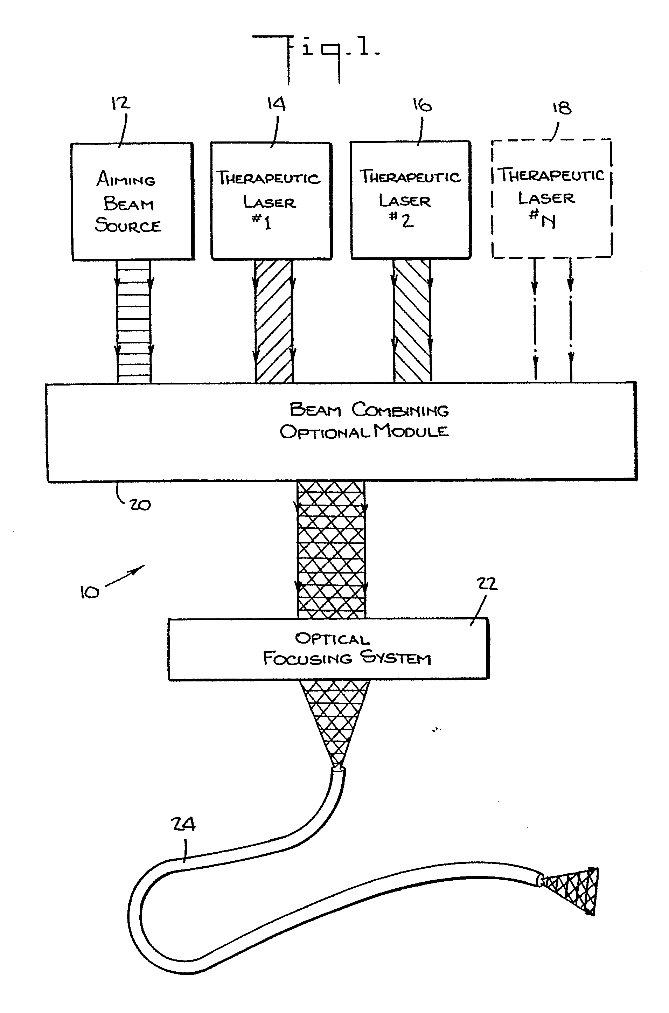

The description herein presented refers to the accompanying drawings in which like reference numerals refer to like parts throughout the several views. Referring to Fig. 1, there is illustrated a schematic representation of medical laser system 10 for transmitting energy to a tissue site. The system includes energy source 12 for emitting visible light or aiming beam and includes at least one energy source 14, 16, 18 for emitting infrared therapeutic radiation. The system will further include beam combining optical module 20 and optical focusing system 22 when more than one therapeutic laser is employed. Specific detail is not provided with respect to beam combining and focusing for such is within the preview of one skilled in the art.

Lastly, depicted in Fig. 1 is laser catheter 24. The catheter is thin and flexible and is especially adaptable for insertion into small, convoluted body vessels or passageways. Not shown in this view is the path of transmission of visible light which will be shown in other figures and which will be delivered through catheter 24.

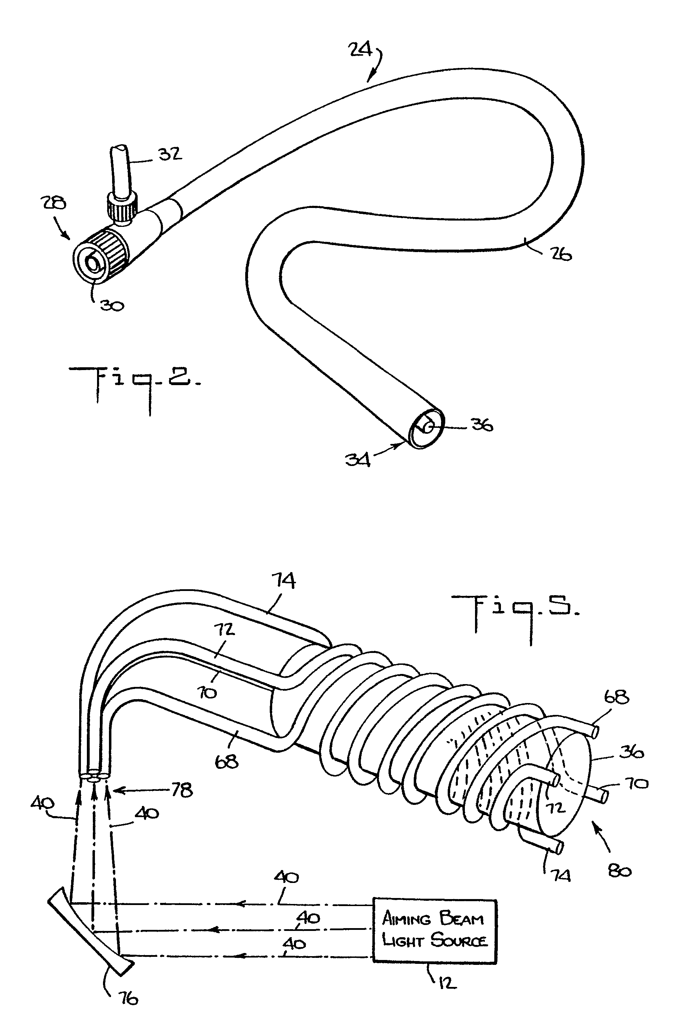

Turning to Fig. 2, there is shown catheter 24 in greater detail. The catheter has a body portion 26 and, at proximal end 28, there is a connector 30 for coupling the catheter to the optical foousing system 22 and energy sources of Fig. 1. Also shown is a gas flow delivery system 32 and, at distal end 34 of catheter 24, there is depicted the end of solid core optical fiber 36 located in the catheter body. The fiber travels substantially the full length of the catheter. Typically, the composition of fiber 36 might be fiber selected from the metal halide group of fibers. A fiber could be selected from, but not limited to, the group consisting of chalcogenide, sapphire, heavy metal fluoride, metal halide crystal, silica and non-oxide glasses. Also typically, fiber 36 would preferably have a diameter in a range of from about 200 to about 600 microns. Connector 30 is a conventional coupling means and gas flow delivery, from a source not shown, is provided for cooling purposes at the distal or energy exit end of the fiber.

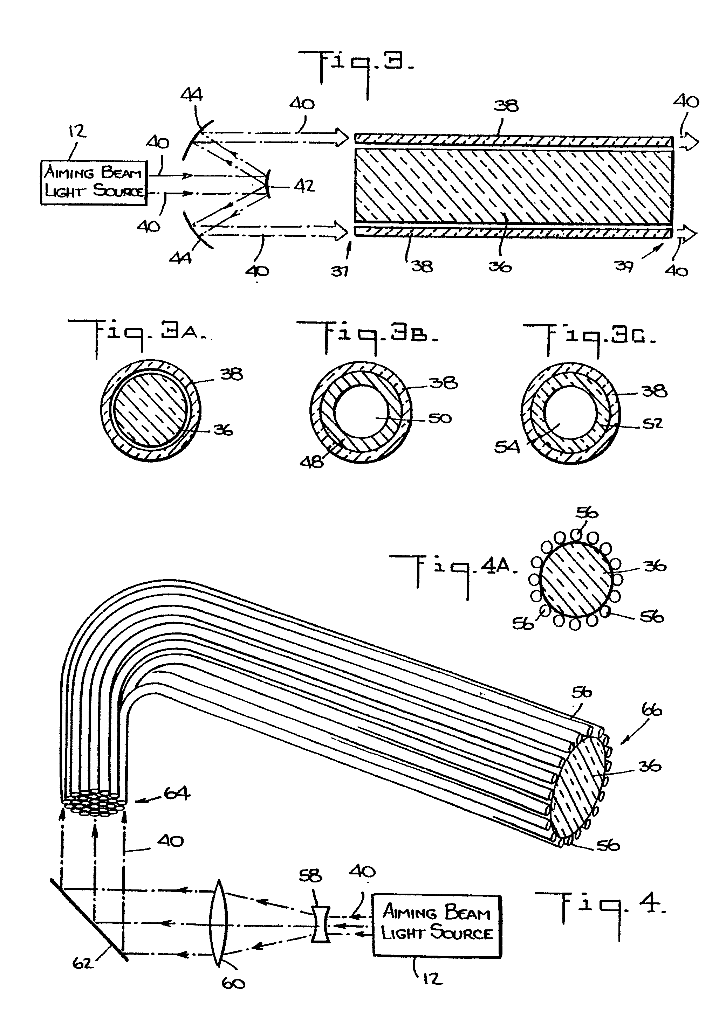

Fig. 3 depicts one embodiment of the invention showing an enlarged partial sectional view of fiber 36 located in catheter 24. In this view the catheter body is not shown. As can be seen in this view, schematically depicted is visible light source 12 and and its transmission path. This embodiment employs a light transmissive hollow tube or sleeve 38 which surrounds and encompasses fiber 36. Visible light or aiming beam, designated 40, is shown leaving source 12, reflected by a first mirror 42 to annular mirror 44, and delivered to a first end of sleeve 38, the location of which is generally designated 37, for transmission therethrough to exit at a second end thereof, the location of which is generally designated 39, for delivery to a tissue site (not shown). Sleeve 38, typically, would be quartz or glass but other light transmissive materials would be suitable. Visible light 40 can be coupled to the first end 37 of sleeve 38 using a Cassegrain type telescope or other equivalent means. Although not shown in this view, infrared therapeutic radiation is conveyed from a laser source or sources (14, 16, 18) and through solid core fiber 36. The visible light beam 40 exiting end 39 of sleeve 38 is annular in nature and completely surrounds the infrared therapeutic laser beam exiting the end of fiber 36. The ends of sleeve 38 and fiber 36 are shown to be coterminous but this may vary somewhat so long as the ends are substantially coterminous. Fig. 3A shows an elevational view of end 39. A hollow core optical fiber 48 with air core 50, as shown in Fig. 3B, could replace solid core optical fiber 36. The composition of fiber 48 could be as heretofore specified in respect to fiber 36. The remaining structure would be as shown in Fig. 3. A metal waveguide 52 having air core 54, as shown in Fig. 3C, could also be used to replace fiber 36. Likewise, the remaining structure would be as set forth in Fig. 3. The flexibility of the optical assembly set forth in Fig. 3 will be limited by the mechanical properties of sleeve 38. The embodiment of Fig. 3 would be appropriate for those systems in which minor catheter flexure is required.

Turning next to Fig. 4, there is depicted another embodiment of the invention. Here, the structure consists of solid core optical fiber 36 surrounded by a number of concentrically oriented individual small diameter rods or fibers 56 which are arranged parallel to one another and parallel to the longitudinal axis of fiber 36. The diameter of the rods or fibers 56 may vary but preferably the diameter thereof ranges from about 10 to about 100 microns. Visible light or aiming beam 40 is shown leaving source 12, passing through defocusing lens 58 and converging lens 60, reflected by mirror 62 and delivered to a first end of gathered optical rods or optical fibers 56, the location of which is generally designated 64, for passage therethrough to exit at a second end thereof, the location of which is generally designated 66, for delivery to a tissue site (not shown). Here, as before, the ends of rods and fiber are shown to be coterminous at 66 but, again, as before, this may vary slightly so long as the ends are substantially coterminous. In this view, there is shown solid core optical fiber 36 but, as heretofore indicated with respect to the Fig. 3 embodiment, hollow core optical fiber 48 and metal waveguide 52 could replace fiber 36. Likewise, the material composition of the inner fiber and the outer rods or fibers would be as previously set forth. Because of the greater inherent flexibility of individual rods or fibers 56 as compared to sleeve 38 of Fig. 3, this embodiment and its modified configurations will allow the infrared component to be flexed through smaller radii of curvature arcs. Moreover, the bundling of rods or fibers 56 in a generally circular cross-section at end 64 facilitate coupling of visible light 40 into the aiming beam external sheath for light delivery to end 66.

Lastly, turning to Fig. 5, there is illustrated yet another embodiment of the invention. Here, the structure consists of solid core optical fiber 36 surrounded by a number of individual fibers 68, 70, 72 and 74. This embodiment will be more appropriate for those applications in which even greater catheter flexibility is required, namely, for bending into arcs having small radii of curvature. In order to avoid breakage of individual fibers (68, 70, 72, 74) due to severe localized flexure of fiber 36, multiple strands of small diameter fibers 68, 70, 72, 74 are wrapped around fiber 36 in a generally coil-like configuration. The diameter of each of fibers 68, 70, 72 and 74 may be equal or may vary but preferably the diameter or diameters thereof fall in a range of from about 10 to about 100 microns. Although the configuration depicted in Fig. 5 only shows four wrapped fiber strands, a larger number of fiber strands could be utilized to form a complete annulus of fibers encircling fiber 36 much like the encircling shown in Fig. 4. Visible light or aiming beam 40 is shown leaving light energy source 12, reflected by mirror 76 and delivered to a first end of gathered optical fibers (68, 70, 72, 74), the location of which is generally designated 78, for passage therethrough to exit at a second end thereof, the location of which is generally designated 80, for delivery to a tissue site (not shown). As with the last preceding embodiment, the fibers can be pulled together at end 78 to form a bundle of circular cross-section into which the visible aiming beam can be easily coupled. The ends of fiber 36 and fibers 68, 70, 72, 74 are not shown to be coterminous at end 80 but they are substantially coterminous. As before, fiber 36 may be replaced by hollow core optical fiber 48, metal waveguide 52, or equivalent means. Material composition may be as heretofore specified in respect to the visible light conveying fibers and to the infrared radiation conveying fiber. It should be understood that throughout where solid core optical fiber 36 and hollow core optical fiber 48 is shown singularly, equally applicable would be the use of a number of fibers surrounded by the visible light conveying means.

In addition to, providing an annular visible aiming beam, the coil of optical fiber also provides additional mechanical protection for the infrared fiber and serves as a built-in safety system to alert a physician using the catheter in the case of optical delivery system failure. In the event that the infrared fiber breaks, the escaping high power laser energy will melt the visible aiming beam conveying optical fiber before exiting the catheter. This will be immediately observed as a distortion of the annular aiming beam at the tissue site and alert the physician to abort the procedure.

The present invention has been described herein with specific reference to the preferred embodiments thereof. However, those skilled in the art will understand that changes may be made in the form of the invention covered by the claims without departing from the scope and spirit thereof, and that certain features of the invention may sometimes be used to an advantage without corresponding use of the other features.

**Claims**

1. A medical instrument for transmitting energy to a tissue site comprising a first means capable of conveying infra-red laser radiation to said site and a second means capable of conveying visible light to said site, characterised in that said second means is disposed peripherally about said first means and is adapted so that in use said visible light illuminates the tissue site contacted by said laser radiation.

2. A medical instrument for transmitting energy to a tissue site comprising a first energy source (14) (16) (18) for emitting infrared laser radiation and a second energy source (12) for emitting visible light, said instrument including first means operatively associated with said first energy source for conveying said laser radiation to said tissue site and second means operatively associated with said second energy source for conveying said visible light to said tissue site, characterized by said second means being disposed peripherally about said first means, with said visible light adapted to illuminate the tissue site contacted by said laser radiation.

3. The instrument according to claim 1 or 2 wherein said first means comprises at least one flexible optical fiber (36) and said second means comprises at least one optical fiber (56)(68)(70)(72)(74).

4. The instrument according to claim 3 wherein said fiber (68)(70)(72)(74) of said second means is wrapped about said fiber (36) of said first means in a generally coil-like configuration.

5. The instrument according to claim 3 wherein said second means comprises a plurality of optical fibers (S6) oriented substantially parallel to one another.

6. The instrument according to claim 1 or 2 wherein said first means comprises at least one flexible optical fiber (36) and said second means comprises one or more flexible elongated rods (56), with said rods being disposed substantially parallel to one another when said second means comprises a plurality of rods.

7. The instrument according to claim 1 or 2 wherein said first means comprises at least one flexible optical fiber (36) and said second means comprises a light transmissive sleeve (38).

8. The instrument according to claim 1 or 2 wherein said first means comprises a metal waveguide (52) and said second means comprises a light transmissive sleeve (38).

9. The instrument according to claim 1 or 2 wherein said first means comprises a metal waveguide (52) and said second means comprises at least one flexible optical fiber (56)-(68)(70)(72)(74).

10. The instrument according to claim 9 wherein said fiber (68)(70)(72)(74) is wrapped about said waveguide (52) in a generally coil-like configuration.

11. The instrument according to claim 1 or 2 wherein said first means comprises a metal waveguide (52) and said second means comprises one or more flexible elongated rods (56), with said rods being disposed substantially parallel to one another when said second means comprises a plurality of rods.

Fig.1.

Fig.2.

Fig.5.

AIMING BEAM
LIGHT SOURCE

Fig.3.

Fig.3A.

Fig.3B.

Fig.3C.

Fig.4A.

Fig.4.